# EUROPEAN PATENT APPLICATION

(11) **EP 1 611 888 A1**
(43) Date of publication of application: **04.01.2006**
(21) Application number: 04724713.5
(22) Date of filing: 31.03.2004
(51) Int. Cl.: A61K 31/381, C07D 495/04, A61P 11/14

(54) **ANTITUSSIVES**

(30) Priority: 31.03.2003 JP 2003094506
(71) Applicant: KYOWA HAKKO KOGYO CO., LTD., Chiyoda-ku, Tokyo 100-8185 (JP)
(72) Inventor: MIKI, Ichiro. c/oPharmaceutical Research Center, Nagaizumi-cho, Sunto-Gun. Shizuoka 411-8 (JP); ISHII, Hidee, Shizuoka 411-2114 (JP)
(74) Representative: Vossius & Partner
(86) International application number: PCT/JP2004/004578
(87) International publication number: WO 2004/087131

(57) **Abstract**

An antitussive which comprises, as an active ingredient, a tricyclic compound represented by Formula (I) 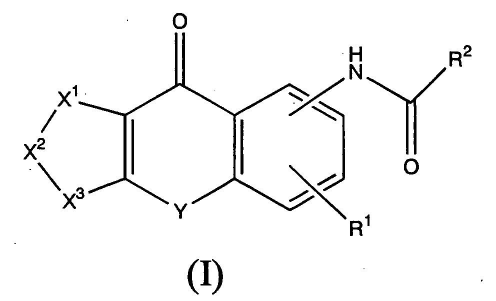
[wherein R¹ represents a hydrogen atom, substituted or unsubstituted lower alkyl or the like,
X¹-X²-X³ represents CR⁵=CR⁶-CR⁷=CR⁸ (wherein R⁵, R⁶, R⁷ and R⁸ may be the same or different and each represents a hydrogen atom, substituted or unsubstituted lower alkyl or the like,
Y represents -CH₂S- or the like, and
R² represents a hydrogen atom, substituted or unsubstituted lower alkyl or the like] or a pharmaceutically acceptable salt thereof.

## Description

### Technical Field

The present invention relates to an antitussive which comprises, as an active ingredient, a tricyclic compound or a pharmaceutically acceptable salt thereof.

### Background Art

Cough plays an important role in airway clearance in host defense such as mucociliary clearance, expectoration, etc. With regard to induction of cough in animals, mechanical stimulation, stimulation by citric acid, capsaicin and substance P, etc. have been known [*Nippon Yakurigaku Zasshi,* volume 105, page 41 (1995)].

As to an antitussive for various kinds of respiratory diseases, codeine phosphate which is a central antitussive has been often used. However, in codeine phosphate, many adverse effects such as constipation, increase in viscosity of sputum, anorexia, withdrawal symptoms including nausea, emesis, diarrhea, abdominal pain, mydriasis, headache, insomnia, anxiety, delirium, tremor, respiratory distress, or the like, respiratory depression, etc. have been known. Although dextromethorphan,benproperine phosphate,dimemorfan phosphate, tipepidine hibenzate, eprazinone hydrochloride, etc. have been used as a non-narcotic antitussive, it has been known that they also have many adverse effects such as drowsiness, insomnia, vertigo, excitation, anorexia, constipation, abdominal pain, dry mouth, exanthema and itch. Further, as an antitussive in bronchial asthma, chronic bronchitis, pulmonary emphysema, diffuse panbronchiolitis, etc., there has been used a bronchodilator such as theophylline, procaterol hydrochloride, clenbuterol hydrochloride, or the like, which directly acts on bronchi to relax the bronchial smooth muscle. However, in theophylline, adverse effects such as convulsion, disturbance of consciousness, acute encephalopathy, rhabdomyolysis, hematemesis, tachypnea, hyperglycemia, etc. have been known while, in procaterol hydrochloride and clenbuterol hydrochloride, adverse effects such as severe reduced level of serum potassium, hypersensitivity including exanthema, itch, etc., psychoneural symptoms including tremor, headache, vertigo, etc., cardiovascular events including palpitation, tachycardia, hot flash, etc., gastrointestinal symptoms including emesis, dry mouth, gastric dysphoria, etc., and hepatic function disturbance including rises of glutamic-oxaloacetic transaminase (GOT) and glutamic-pyruvic transaminase (GPT), and the like have been known. In case where an excessive use of drug is continued, there is a risk of arrhythmia or cardiac arrest [*Kokyu*, volume 3, page 924 (1984)]. Under such circumstances, there has been a demand for antitussive having no adverse effects and being able to be used for many diseases.

In the meanwhile, it has been known that a compound having the same structure as the compound used in the present invention has an action of extending the urination interval which is noted when the bladder is filled and is useful for treatment or improvement of pollakiuria, urinary incontinence, feeling of urgency of urination, feeling of residual urine, etc. in various diseases or symptoms including neurogenic bladder, unstable bladder or the like (WO 97/14672; WO 98/46587).

### Disclosure of the Invention

An object of the present invention is to provide an antitussive which comprises, as an active ingredient, a tricyclic compound or a pharmaceutically acceptable salt thereof.

The present invention relates to (1)-(27).
(1) An antitussive which comprises, as an active ingredient, a tricyclic compound represented by Formula (I) 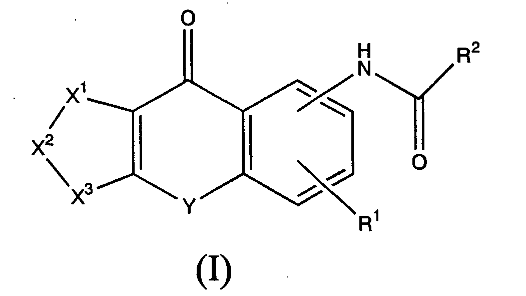
   (wherein R¹ represents a hydrogen atom, substituted or unsubstituted lower alkyl, substituted or unsubstituted lower alkoxy or halogen,
   X¹-X²-X³ represents CR⁵=CR⁶-CR⁷=CR⁸ [wherein R⁵, R⁶, R⁷ and R⁸ may be the same or different and each represents a hydrogen atom, substituted or unsubstituted lower alkyl, hydroxy, substituted or unsubstituted lower alkoxy, nitro, amino, mono(lower alkyl)-substituted amino, di(lower alkyl)-substituted amino, substituted or unsubstituted lower alkanoylamino or halogen], N(O)ₘ=CR⁶-CR⁷=CR⁸ (wherein R⁶, R⁷ and R⁸ have the same meanings as defined above, respectively and m represents 0 or 1), CR⁵=CR⁶-N(O)ₘ=CR⁸ (wherein R⁵, R⁶, R⁸ and m have the same meanings as defined above, respectively), CR⁵=CR⁶-CR⁷=N(O)ₘ (wherein R⁵, R⁶, R⁷ and m have the same meanings as defined above, respectively), CR⁵=CR⁶-O (wherein R⁵ and R⁶ have the same meanings as defined above, respectively), CR⁵=CR⁶-S (wherein R⁵ and R⁶ have the same meanings as defined above, respectively), O-CR⁷=CR⁸ (wherein R⁷ and R⁸ have the same meanings as defined above, respectively), S-CR⁷=CR⁸ (wherein R⁷ and R⁸ have the same meanings as defined above, respectively) or O-CR⁷=N (wherein R⁷ has the same meaning as defined above),
   Y represents -CH₂S-, -CH₂SO-, -CH₂SO₂-, -CH₂O-, -CH=CH-, -(CH₂)ₚ-(wherein p represents an integer of 0 to 2), -SCH₂-, -SOCH₂-, -SO₂CH₂- or -OCH₂-, and
   R² represents a hydrogen atom, substituted or unsubstituted lower alkyl, substituted or unsubstituted lower alkenyl, substituted or unsubstituted lower alkoxy, amino, mono(substituted or unsubstituted lower alkyl)-substituted amino, di(substituted or unsubstituted lower alkyl)-substituted amino, substituted or unsubstituted aryl, substituted or unsubstituted heteroaryl, substituted or unsubstituted aralkylamino, substituted or unsubstituted arylamino, or a substituted or unsubstituted heterocyclic group} or a pharmaceutically acceptable salt thereof.
(2) An antitussive which comprises, as an active ingredient, a tricyclic compound represented by Formula (Ia) 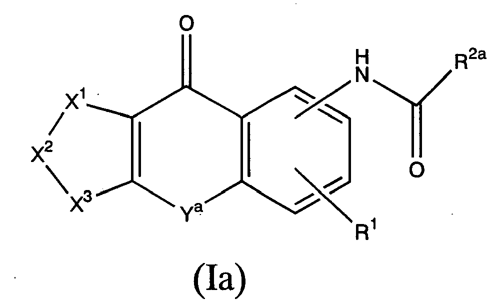
   [wherein R¹ and X¹-X²-X³ have the same meanings as defined above, respectively,
   Y^{a} represents -CH₂SO₂-, -SCH₂-, -SOCH₂-, -SO₂CH₂- or -OCH₂- and when Y^{a} is -CH₂SO₂-, -SCH₂-, -SOCH₂- or -SO₂CH₂-,
   R^{2a} represents a hydrogen atom, substituted or unsubstituted lower alkyl, substituted or unsubstituted lower alkenyl, substituted or unsubstituted lower alkoxy, amino, mono(substituted or unsubstituted lower alkyl)-substituted amino, di(substituted or unsubstituted lower alkyl)-substituted amino, substituted or unsubstituted aryl, substituted or unsubstituted heteroaryl, substituted or unsubstituted aralkylamino, substituted or unsubstituted arylamino, a substituted or unsubstituted heteroalicyclic group, or a substituted or unsubstituted nitrogen-containing heterocyclic group and
   when Y^{a} is -OCH₂-,
   R^{2a} represents a hydrogen atom, trifluoromethyl, substituted or unsubstituted lower alkenyl, substituted or unsubstituted lower alkoxy, amino, mono(substituted or unsubstituted lower alkyl)-substituted amino, di(substituted or unsubstituted lower alkyl)-substituted amino, substituted or unsubstituted aryl, substituted or unsubstituted heteroaryl, substituted or unsubstituted aralkylamino, substituted or unsubstituted arylamino, a substituted or unsubstituted heteroalicyclic group, a substituted or unsubstituted nitrogen-containing heterocyclic group, or Formula (II) 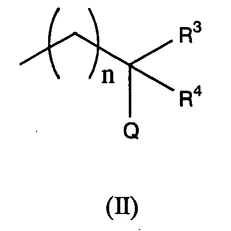
   (wherein n is 0 or 1; R³ and R⁴ may be the same or different and represents a hydrogen atom, substituted or unsubstituted lower alkyl, substituted or unsubstituted cycloalkyl, substituted or unsubstituted aryl, or substituted or unsubstituted aralkyl, or R³ and R⁴ may be combined together with the adjacent carbon atom thereto to form cycloalkyl; and Q represents hydroxy, substituted or unsubstituted lower alkoxy, amino or halogen)] or a pharmaceutically acceptable salt thereof.
(3) The antitussive according to (2), wherein Y^{a} is -CH₂SO₂-, -SCH₂-, -SOCH₂- or -SO₂CH₂-.
(4) The antitussive according to (2), wherein Y^{a} is -OCH₂-.
(5) The antitussive according to any of (2) to (4), wherein R¹ is a hydrogen atom, substituted or unsubstituted lower alkoxy or halogen.
(6) The antitussive according to any of (2) to (4), wherein R¹ is a hydrogen atom.
(7) The antitussive according to any of (2), (5) and (6), wherein Y^{a} is -CH₂SO₂-, -SO₂CH₂- or -OCH₂-.
(8) The antitussive according to any of (2), (5) and (6), wherein Y^{a} is -CH₂SO₂- or -SO₂CH₂-.
(9) The antitussive according to any of (2), (5) and (6), wherein Y^{a} is -CH₂SO₂-.
(10) The antitussive according to any of (2) to (9), wherein X¹-X²-X³ is S-CR⁷=CR⁸ (wherein R⁷ and R⁸ have the same meanings as defined above, respectively).
(11) The antitussive according to any of (2) to (9), wherein X¹-X²-X³ is CR⁵=CR⁶-CR⁷=CR⁸ (wherein R⁵, R⁶, R⁷ and R⁸ have the same meanings as defined above, respectively).
(12) The antitussive according to any of (2) to (11), wherein R^{2a} is Formula (II) 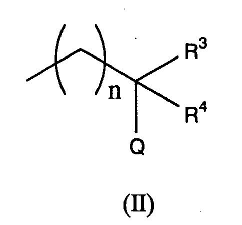
   (wherein n, R³, R⁴ and Q have the same meanings as defined above, respectively).
(13) The antitussive according to (12), wherein n is 0.
(14) The antitussive according to (13), wherein R³ is methyl, R⁴ is trifluoromethyl, and Q is hydroxy.
(15) The antitussive according to (2), wherein R¹ is a hydrogen atom, Y^{a} is -CH₂SO₂-, X¹-X²-X³ is S-CR⁷=CR⁸ (wherein R⁷ and R⁸ have the same meanings as defined above, respectively), and R² is Formula (III) 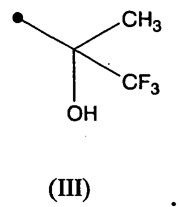
(16) An antitussive which comprises, as an active ingredient, a tricyclic compound represented by Formula (Ib) 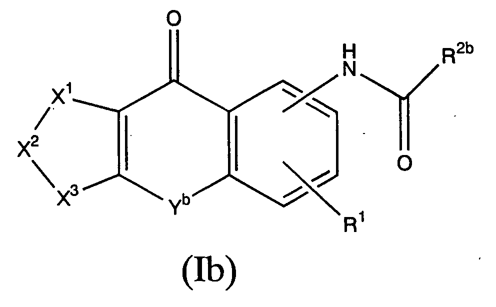
   [wherein R¹ and X¹-X²-X³ have the same meanings as defined above, respectively,
   Y^{b} represents -CH₂O-, -CH₂S-, -CH₂SO-, -CH=CH- or -(CH₂)_{P}-(wherein p has the same meaning as defined above) and
   R^{2b} represents Formula (III) 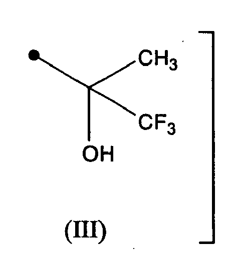
   or a pharmaceutically acceptable salt thereof.
(17) The antitussive according to (16), wherein X¹-X²-X³ is CR⁵=CR⁶-CR⁷=CR⁸ (wherein R⁵, R⁶, R⁷ and R⁸ have the same meanings as defined above, respectively) or CR⁵=CR⁶-CR⁷=N (wherein R⁵, R⁶ and R⁷ have the same meanings as defined above, respectively).
(18) The antitussive according to (16), wherein X¹-X²-X³ is CR⁵=CR⁶-O (wherein R⁵ and R⁶ have the same meanings as defined above, respectively) or CR⁵=CR⁶-S (wherein R⁵ and R⁶ have the same meanings as defined above, respectively).
(19) The antitussive according to (16), wherein X¹-X²-X³ is O-CR⁷=CR⁸ (wherein R⁷ and R⁸ have the same meanings as defined above, respectively) or S-CR⁷=CR⁸ (wherein R⁷ and R⁸ have the same meanings as defined above, respectively).
(20) The antitussive according to any of (16) to (19), wherein Y^{b} is -CH₂O-.
(21) The antitussive according to any of (16) to (19), wherein Y^{b} is -(CH₂)ₚ- (wherein p has the same meaning as defined above).
(22) The antitussive according to (21), wherein p is 0.
(23) The antitussive according to (21), wherein p is 2.
(24) The antitussive according to any of (16) to (19), wherein Y^{b} is -CH=CH-.
(25) The antitussive according to any of (16) to (19), wherein Y^{b} is -CH₂S- or -CH₂SO-.
(26) A method for alleviation of a cough, which comprises a step of administering an effective amount of the tricyclic compound or the pharmaceutically acceptable salt thereof described in any of (1) to (25).
(27) Use of the tricyclic compound or the pharmaceutically acceptable salt thereof described in any of (1) to (25) for the manufacture of an antitussive.

Hereinafter, the compounds represented by Formula (I) are referred to as Compounds (I), and the same applies to the compounds of other formula numbers.

In the definitions of the groups in Formula (I), the lower alkyl includes, for example, straight-chain or branched lower alkyl having 1 to 8 carbon atoms, more specifically, methyl, ethyl, propyl, isopropyl, butyl, isobutyl, sec-butyl, tert-butyl, pentyl, hexyl, 1,2,2-trimethylpropyl, heptyl, octyl, etc.

The halogen means fluorine, chlorine, bromine and iodine atoms.

The lower alkyl moiety of the lower alkoxy, mono(lower alkyl)-substituted amino and di(lower alkyl)-substituted amino has the same meaning as the lower alkyl defined above.

The lower alkanoyl moiety of the lower alkanoylamino includes, for example, alkanoy having 1 to 6 carbon atoms, more specifically, formyl, acetyl, propanoyl, butanoyl, pentanoyl, 2,2-dimethylpropanoyl, hexanoyl, etc.

The lower alkenyl includes, for example, straight-chain or branched lower alkenyl having 2 to 6 carbon atoms, more specifically, vinyl, allyl, 1-propenyl, methacryl, 1-butenyl, crotyl, pentenyl, hexenyl, etc.

The aryl and the aryl moiety of the arylamino include, for example, phenyl, naphthyl, etc, and the heteroaryl includes, for example, pyridyl, furyl, thienyl, quinolyl, imidazolyl, benzimidazolyl, thiazolyl, etc.

The aralkyl moiety of the aralkylamino includes, for example, aralkyl having 7 to 12 carbon atoms, more specifically, benzyl, phenethyl, naphthylmethyl, etc.

The heterocyclic group includes, for example, heteroalicyclic groups, nitrogen-containing heterocyclic groups, etc. The heteroalicyclic group includes, for example, tetrahydrofuryl, tetrahydrothienyl, chromanyl, etc. The nitrogen-containing heterocyclic group includes, for example, heterocyclic groups containing 1 or 2 nitrogen atoms in the ring and optionally containing other hetero atoms such as oxygen, sulfur, etc. and more specifically, it includes pyrrolidinyl, pipecolinyl, piperazinyl, piperidinyl, morpholinyl, thiomorpholinyl, oxazolyl, etc.

The substituted lower alkyl, the substituted lower alkoxy, the mono(substituted lower alkyl)-substituted amino, the di (substituted lower alkyl)-substituted amino, the substituted lower alkanoylamino and the substituted lower alkenyl each have substituents of 1 to a substitutable number (preferably 1 to 6, more preferably 1 to 4) which substituents are the same or different. Examples of the substituents include hydroxy, halogen, nitro, amino, mono(lower alkyl)-substituted amino, di(lower alkyl)-substituted amino, cycloalkyl, substituted cycloalkyl [the substituted cycloalkyl has 1 to 3 substituents which are the same or different, such as hydroxy, halogen, nitro, amino, mono(lower alkyl)-substituted amino, di(lower alkyl)-substituted amino, lower alkoxy, etc], aryl, substituted aryl (the substituent in the substituted aryl has the same meaning as that in the substituted aryl defined below), aralkyl, substituted aralkyl (the substituent in the substituted aralkyl has the same meaning as that in the substituted aralkyl defined below), lower alkoxy, substituted lower alkoxy [the substituted lower alkoxyhas 1 to 3 substituents which are the same or different, such as hydroxy, halogen, nitro, amino, mono(lower alkyl)-substituted amino, di(lower alkyl)-substituted amino, lower alkoxy, etc]. Further, the two lower alkyl moieties on the same carbon atom of the substituted lower alkyl may form, together with the said carbon atom, an aliphatic ring. When the substituted lower alkyl is substituted methyl or substituted ethyl, the number of the substituents may be, for example, 1 to 3 and the substituents may be the same or different. For example, the substituents include lower alkyl, substituted lower alkyl [the substituted lower alkyl has 1 to 3 substituents which are the same or different, such as hydroxy, halogen, nitro, amino, mono(lower alkyl)-substituted amino, di(lower alkyl)-substituted amino, lower alkoxy, etc], etc.

In the definition of the substituents for the substituted lower alkyl, substituted lower alkoxy, mono (substituted lower alkyl)-substituted amino, di(substituted lower alkyl)-substituted amino, substituted lower alkanoylamino and substituted lower alkenyl, the halogen has the same meaning as defined above, the lower alkyl and the lower alkyl moiety of the mono(lower alkyl)-substituted amino, di(lower alkyl)-substituted amino and lower alkoxy have the same meaning as that of the above-described lower alkyl, and the aryl has the same meaning as defined above. The cycloalkyl and the cycloalkyl moiety of aliphatic rings include, for example, cycloalkyl having 3 to 8 carbon atoms, more specifically, cyclopropyl, cyclobutyl, cyclopentyl, cyclohexyl, cycloheptyl, cyclooctyl, etc. The aralkyl includes, for example, aralkyl having 7 to 12 carbon atoms, more specifically, benzyl, phenethyl, naphthylmethyl, etc.

The substituted aryl, the substituted heteroaryl, the substituted aralkylamino and the substituted arylamino have 1 to 3 substituents which are the same or different, and examples of the substituents include loweralkyl, hydroxy, amino, halogen, etc.

In the definition of the substituents in the substituted aryl, substituted heteroaryl, substituted aralkylamino and substituted arylamino, the lower alkyl and halogen have the same meanings as defined above, respectively.

The substituted heterocyclic group has 1 to 3 substituents which are the same or different, and examples of the substituents include lower alkyl, hydroxy, halogen, etc.

In the definition of the substituents in the substituted heterocyclic group, the lower alkyl and halogen have the same meanings as defined above, respectively.

In the definitions of formula (Ia) and formula (Ib), the lower alkyl includes, for example, straight-chain or branched lower alkyl having 1 to 6 carbon atoms, more specifically, methyl, ethyl, propyl, isopropyl, butyl, isobutyl, sec-butyl, tert-butyl, pentyl, hexyl, 1,2,2-trimethylpropyl, etc.

The halogen means fluorine, chlorine, bromine and iodine atoms.

The lower alkyl moiety of the lower alkoxy, mono(lower alkyl)-substituted amino and di(lower alkyl)-substituted amino has the same meaning as that of the lower alkyl defined above.

The lower alkenyl includes, for example, straight-chain or branched lower alkenyl having 2 to 6 carbon atoms, more specifically, vinyl, allyl, 1-propenyl, methacryl, 1-butenyl, crotyl, pentenyl, hexenyl, etc.

The aryl and the aryl moiety of the arylamino include, for example, phenyl, naphthyl, etc, and the heteroaryl includes, for example, pyridyl, furyl, thienyl, quinolyl, imidazolyl, benzimidazolyl, thiazolyl, etc.

The aralkyl and the aralkyl moiety of the aralkylamino include, for example, aralkyl having 7 to 12 carbon atoms, more specifically, benzyl, phenethyl, naphthylmethyl, etc.

The heteroalicyclic group includes, for example, tetrahydrofuryl, tetrahydrothienyl, chromanyl, etc. The nitrogen-containing heterocyclic group includes, for example, a heterocyclic group containing 1 or 2 nitrogen atoms in the ring and optionally containing other hetero atoms such as oxygen , sulfur, etc. in which the nitrogen atom in the ring bonds to the adjacent carbonyl group. For example, it includes pyrrolidinyl, pipecolinyl, piperazinyl, piperidinyl, morpholinyl, thiomorpholinyl, oxazolyl, etc.

The cycloalkyl includes, for example, cycloalkyl having 3 to 8 carbon atoms, more specifically, cyclopropyl, cyclobutyl, cyclopentyl, cyclohexyl, cycloheptyl, cyclooctyl, etc.

The substituted lower alkyl, the substituted lower alkoxy, the mono(substituted lower alkyl)-substituted amino, the di (substituted lower alkyl)-substituted amino, the substituted lower alkenyl and the substituted cycloalkyl have 1 to 3 substituents which are the same or different. Examples of the substituents include hydroxy, halogen, nitro, amino, mono(lower alkyl)-substituted amino, di(lower alkyl)-substituted amino, lower alkoxy, etc. When the substituted lower alkyl is substituted methyl or substituted ethyl, the number of the substituents may be, for example, 1 to 3 and the substituents may be the same or different. For example, the substituents include lower alkyl, substituted lower alkyl [the substituted lower alkyl has 1 to 3 substituents which are the same or different, such as hydroxy, halogen, nitro, amino, mono(lower alkyl)-substituted amino, di(lower alkyl)-substituted amino, lower alkoxy, etc], cycloalkyl, substituted cycloalkyl [the substituted cycloalkyl has 1 to 3 substituents which are the same or different, such as hydroxy, halogen, nitro, amino, mono(lower alkyl)-substituted amino, di(lower alkyl)-substituted amino, lower alkoxy, etc], aryl, substituted aryl [the substituted aryl has 1 to 3 substituents which are the same or different, such as hydroxy, halogen, nitro, amino, mono(lower alkyl)-substituted amino, di(lower alkyl)-substituted amino, lower alkoxy, etc], aralkyl, substituted aralkyl [the substituted aralkyl has 1 to 3 substituents which are the same or different, such as hydroxy, halogen, nitro, amino, mono(lower alkyl)-substituted amino, di(lower alkyl)-substituted amino, lower alkoxy, etc], substituted lower alkoxy [the substituted lower alkoxy has 1 to 3 substituents which are the same or different, such as hydroxy, halogen, nitro, amino, mono(lower alkyl)-substituted amino, di(lower alkyl)-substituted amino, lower alkoxy, etc]. Further, the two lower alkyl on the same carbon atom of the substituted methyl or the substituted ethyl may form, together with the said carbon atom, an aliphatic ring.

In the definition of the substituents of the substituted lower alkyl, substituted lower alkoxy, mono (substituted lower alkyl)-substituted amino, di(substituted lower alkyl)-substituted amino, substituted lower alkenyl and substituted cycloalkyl, the halogen, the cycloalkyl, the cycloalkyl moiety of the aliphatic ring, the aryl and the aralkyl have the same meanings as defined above, respectively. The lower alkyl and the lower alkyl moiety of the mono(lower alkyl)-substituted amino, di(lower alkyl)-substituted amino and lower alkoxy have the same meaning as that of the lower alkyl defined above.

The substituted aryl, the substituted heteroaryl, the substituted aralkyl, the substituted aralkylamino and the substituted arylamino have 1 to 3 substituents which are the same or different. Examples of the substituents include lower alkyl, hydroxy, amino, halogen, etc.

In the definition of the substituents of the substituted aryl, substituted heteroaryl, substituted aralkyl, substituted aralkylamino and substituted arylamino, the lower alkyl and the halogen have the same meanings as defined above, respectively.

The substituted heteroalicyclic group and the substituted nitrogen-containing heterocyclic group have 1 to 3 substituents which are the same or different. Examples of the substituents include lower alkyl, hydroxy, halogen, etc.

In the definition of the substituents in the substituted heteroalicyclic group and the substituted nitrogen-containing heterocyclic group, the lower alkyl and the halogen have the same meanings as defined above, respectively.

The pharmaceutically acceptable salts of Compound (I), Compound (Ia) and Compound (Ib) include pharmaceutically acceptable acid addition salts, for example, inorganic acid addition salts such as hydrochloride, hydrobromide, hydroiodide, nitrate, sulfate, phosphate, etc. and organic acid addition salts such as formate, acetate, benzoate, maleate, fumarate, succinate, tartrate, citrate, oxalate, glyoxylate, aspartate, methanesulfonate, ethanesulfonate, benzenesulfonate, etc.

The compounds used in the present invention can be produced according to the methods disclosed in the above publications or similar methods, and can be isolated and purified by purification methods conventionally used in synthetic organic chemistry, such as neutralization, filtration, extraction, washing, drying, concentration, recrystallization, various kinds of chromatography, etc.

When it is desired to obtain a salt of the compound used in the present invention, in the case where it is produced in the form of the salt, it can be subjected to purification as such, and where it is produced in the form of a free base, it can be converted into a salt, after being dissolved or suspended in a suitable solvent, by adding an acid thereto.

There may be optical isomers for some of the compounds used in the present invention. All possible stereoisomers and mixtures thereof can be used as active ingredients of the antitussive of the present invention. Further, all possible stereoisomers and mixtures thereof described above can be used as active ingredients of the agent for alleviation of sneeze of the present invention.

The compounds or pharmaceutically acceptable salts thereof used in the present invention may exist in the form of adducts with water or various solvents, which can also be used as active ingredients of the antitussive of the present invention. Further, the adducts described above can be used as active ingredients of the agent for alleviation of sneeze of the present invention.

Antitussive activity is able to be evaluated by suppression of the cough induced by inhalation of a substance such as citric acid, capsaicin, etc. It is also possible to evaluate antitussive activity by suppression of the cough induced by a direct infusion of citric acid or capsaicin into airway [*Nippon Yakurigaku Zasshi,* volume 120, page 237 (2002)]. It is further possible to evaluate antitussive activity by suppression of the cough by a physical stimulation, for example, contact stimulation of throat or airway [*Eur. J. Pharmacol.,* volume 329, page 93 (1997)].

Now, pharmacological action of the representative Compound (I) will be more specifically illustrated by way of a Test Example. As to the test compound, (S)-(+)-3,3,3-trifluoro-2-hydroxy-2-methyl-N-(5,5,10-trioxo-4,10-dihydro-thieno[3,2-c][1]benzothiepin-9-yl)propaneamide and 2-hydroxy-2-methyl-N-(5,5,10-trioxo-4,10-dihydrothieno[3,2-c][1]benzothiepin-9-yl)propaneamide were used. In this specification, the above two compounds will be referred to as Compound 1 and Compound 2, respectively. Incidentally, the Compound 1 and Compound 2 are the same as Compound (1-25) and Compound (3-12) in WO 98/46587, respectively.

Test Example 1: Evaluation using the citric acid-induced cough model of guinea pig

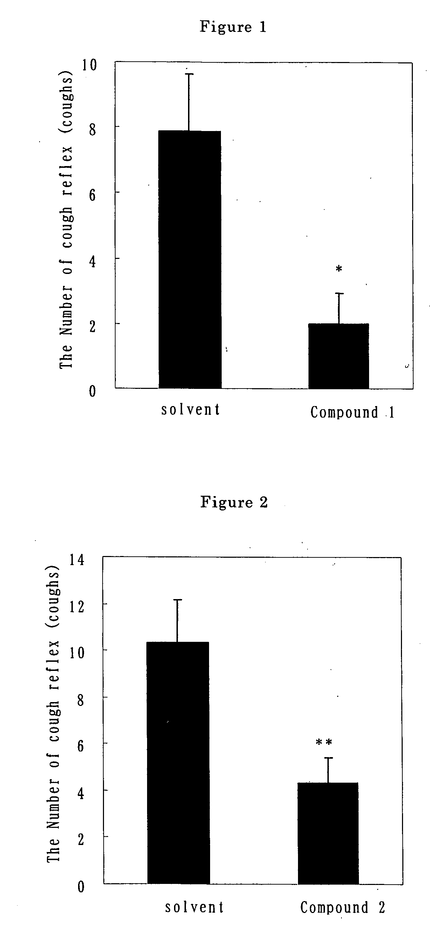

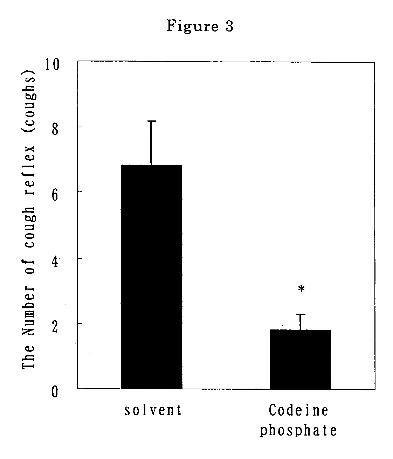

Guinea pigs (Hartley strain; male; six weeks age; Nippon SLC) were used for the test. Induction of cough reflex by citric acid was conducted in a similar manner to the method by Hosoe, et al. [*Yakuri to Rinsho,* volume 5, page 2147 (1995)]. Thus, the guinea pig was fixed in a double chamber plethysmograph box (manufactured by Buxco Electronics) for guinea pigs and ventilation was conducted at about 1.6 L/minute. A physiological saline containing 0.5 mol/L of citric acid (manufactured by Wako Pure Chemical) was sprayed for 2 minutes from the upper part of the box of the head side using an ultrasonic nebulizer (NE-U12 manufactured by Omron) and discharged from the side drain. The cough reflex was measured by the generated cough sound and the respiration waveform. Numbers of coughs generated during 15 minutes from initiation of inhalation of citric acid were counted. Compound 1, Compound 2 and codeine phosphate (manufactured by Shionogi) each was suspended in 0.5 w/v% methyl cellulose 400 cP (manufactured by Wako Pure Chemical) and orally administered 1 hour before the initiation of the inhalation of citric acid. Incidentally, with regard to the Compound 1 and Compound 2, each 2 mg thereof was suspended in 1 mL of 0.5 w/v% methyl cellulose 400 cP while, with regard to codeine phosphate, 4 mg thereof was suspended in 1 mL of 0.5 w/v% methyl cellulose 400 cP and the suspension was administered at 5 mL per kg body weight. Numbers of the cough reflex by inhalation of citric acid in the group administered with the solvent were 7.9 ± 1.8, while those in the group administered with 10 mg/kg of Compound 1 were 2.0 ± 1.0. In this test, seven guinea pigs were used for the group administered with the solvent and for the group administered with Compound 1, respectively. Numbers of the cough reflex were expressed as the mean value ± standard error. As compared with the numbers of the cough reflex in the group administered with the solvent, a significant suppressive response (p = 0.0132) was noted in the group administered with Compound 1 (Fig. 1). Numbers of the cough reflex by inhalation of citric acid in the group administered with 10 mg/kg of Compound 2 were 4.3 ± 1.0, while those in the group administered with the solvent were 10.3 ± 1.8. In this test, twelve guinea pigs were used for the group administered with the solvent and for the group administered with Compound 2, respectively. Numbers of the cough reflex were expressed as the mean value ± standard error. As compared with the numbers of the cough reflex in the group administered with the solvent, a significant suppressive response (p = 0.0090) was noted in the group administered with Compound 2 (Fig. 2). Numbers of the cough reflex in the group administered with 20 mg/kg of codeine phosphate which is a central antitussive were 1.8 ± 0.5, while those in the group administered with the solvent were 6.8 ± 1.4. In this test, six guinea pigs were used for the group administered with the solvent and for the group administered with codeine phosphate. Numbers of the cough reflex were expressed as the mean value ± standard error. As compared with the numbers of the cough reflex in the group administered with the solvent, a significant suppressive response (p = 0.0123) was noted in the group administered with codeine phosphate (Fig. 3).

A pharmaceutical preparation containing the compound of the present invention is able to contain said compound either solely or as a mixture with any effective ingredient for treatment. Such a pharmaceutical preparation is manufactured according to any method which has been well known in the technical art for pharmaceutical preparations by mixing the active ingredient with one or more pharmaceutically acceptable carrier(s).

With regard to a route for administration, it is desirable to use the most effective one for the treatment and its examples are oral and parenteral administrations such as intra-airway, intravenous one, etc.

Examples of the dosage form are tablets, syrup, inhalant, injection and the like.

Liquid preparation such as syrup which is suitable for oral administration is able to be manufactured using water; saccharide such as sucrose, sorbitol, fructose, etc.; glycol such as polyethylene glycol, propylene glycol, etc.; oil such as sesame oil, olive oil, soybean oil, etc.; antiseptic agent such as p-hydroxybenzoate, etc.; flavor such as strawberry flavor, peppermint, etc.; etc. Tablets, powders, granules, etc. are able to be manufactured using excipients such as lactose, glucose, sucrose, mannitol, etc.; disintegrating agents such as starch, sodium alginate, etc.; lubricants such as magnesium stearate, talc, etc.; binders such as polyvinyl alcohol, hydroxypropyl cellulose, gelatin, etc. ; surfactants such as fatty acid ester, etc.; plasticizers such as glycerol, etc.; etc.

With regard to a preparation suitable as an inhalant, dry powder, that dissolved in a solvent being able to be used for metered dose inhaler (MDI), that dissolved in a solvent being able to be used for spray, that dissolved in a solvent being able to be used for nebulizer, etc. are prepared.

Preparations suitable for injection preferably comprise a sterilized aqueous solution containing an active compound being isotonic to blood of the recepient. For example, in the case of injection, a solution for injection is prepared using a salt solution, a glucose solution, a carrier comprising a mixture of salt solution and glucose solution, etc.

In such a parenteral preparation, it is also possible to add one or more auxiliary component (s) selected from diluents, antiseptics, flavors, excipients, disintegrating agents, lubricants, binders, surfactants, plasticizers, etc. as exemplified for oral preparation.

The dose and administering frequency of the compound used in the present invention vary depending upon dosage form, age and body weight of a patient, nature or degree of severeness of the diseases to be treated, etc. and, usually, in the case of oral administration, 0.01 to 1 g or, preferably, 1 to 150 mg per day is administered to an adult once daily or several times a day. In the case of parenteral administration such as inhalation and intravenous administration, 0.01 to 1 g or, preferably, 0.1 to 100 mg per day is administered to an adult once daily or several times a day. However, the dose and the administering frequency as such vary depending upon the above-mentioned various conditions.

### Brief Description of the Drawings

Fig.1
   Fig. 1 shows an action of Compound 1 (10 mg/kg; oral administration) to citric acid-induced cough in guinea pigs. The longitudinal axis shows numbers of cough reflex induced within 15 minutes by inhalation of citric acid.
   *: p < 0.05 (Student's t-test as compared with a solvent-administered group)
Fig. 2
   Fig. 2 shows an action of Compound 2 (10 mg/kg; oral administration) to citric acid-induced cough in guinea pigs. The longitudinal axis shows numbers of cough reflex induced within 15 minutes by inhalation of citric acid.
   *: p < 0.01 (Student's t-test as compared with a solvent-administered group)
Fig. 3
   Fig. 3 shows an action of the codeine phosphate (20 mg/kg; oral administration) to citric acid-induced cough in guinea pigs. The longitudinal axis shows numbers of cough reflex induced within 15 minutes by inhalation of citric acid.
   *: p < 0.05 (Aspin-Welch's test as compared with a solvent-administered group)

### Best Modes for Carrying Out the Invention

The present invention will be more specifically explained with reference to the following examples. However, the scope of the present invention is not limited to the following examples.

### Example 1: Tablets

Tablets having the following composition were prepared according to a conventional method.

Compound 1 (250 g), mannitol (1598.5 g), sodium starch glycolate (100 g), light silicic acid anhydride (10 g), magnesium stearate (40 g) and yellow iron oxide (1.5 g) were mixed according to a conventional method. The resulting mixture was compressed using a tabletting machine equipped with 8 mm diameter punch and die (Purepress Correct-12, Kikusui Seisakusho Ltd.) to prepare tablets each containing 25 mg of the active ingredient.

| | | |
|---|---|---|
| Formulation | Compound 1 | 25 mg |
| | Mannitol | 159.85 mg |
| | Sodium starch glycolate | 10 mg |
| | Light silicic acid anhydride | 1 mg |
| | Magnesium stearate | 4 mg |
| | Yellow iron oxide | 0.15 mg |
| | | 200 mg |

### Example 2: Tablets

Tablets having the following composition are prepared according to a conventional method.

Compound 2 (250 g), mannitol (1598.5 g), sodium starch glycolate (100 g), light silicic acid anhydride (10 g), magnesium stearate (40 g) and yellow iron oxide (1.5 g) were mixed according to a conventional method. The resulting mixture was compressed using a tabletting machine equipped with 8 mm diameter punch and die (Purepress Correct-12, Kikusui Seisakusho Ltd.) to prepare tablets each containing 25 mg of the active ingredient.

| | | |
|---|---|---|
| Formulation | Compound 2 | 25 mg |
| | Mannitol | 159.85 mg |
| | Sodium starch glycolate | 10 mg |
| | Light silicic acid anhydride | 1 mg |
| | Magnesium stearate | 4 mg |
| | Yellow iron oxide | 0.15 mg |
| | | 200 mg |

### Example 3: Capsules

Capsules having the following composition were prepared according to a conventional method.

Compound 1 (500 g), lactose (300 g), light silicic acid anhydride (100 g) and sodium lauryl sulfate (100 g) were mixed according to a conventional method. The resulting mixture was encapsulated in hard capsules No. 1 (content: 100 mg/capsule) using a capsule filler (LZ-64, Zanasi) to prepare capsules each containing 50 mg of the active ingredient.

| | | |
|---|---|---|
| Formulation | Compound 1 | 50 mg |
| | Lactose | 30 mg |
| | Light silicic acid anhydride | 10 mg |
| | Sodium lauryl sulfate | 10 mg |
| | | 100 mg |

### Example 4: Injection

An injection having the following composition is prepared according to a conventional method.

Compound 1 (1 g) is dissolved in 100 g of purified soybean oil, and 12 g of purified egg yolk lecithin and 25 g of glycerin for injection are added thereto. The resulting mixture is made up to 1000 ml with distilled water for injection, kneaded and emulsified according to a conventional method. The obtained dispersion is aseptically filtered using a 0.2µm disposable membrane filter and aseptically packed in glass vials in 2 ml portions to prepare injections each containing 2 mg of the active ingredient per vial.

| | | |
|---|---|---|
| Formulation | Compound 1 | 2 mg |
| | Purified soybean oil | 200 mg |
| | Purified egg yolk lecithin | 24 mg |
| | Glycerin for injection | 50 mg |
| | Distilled water for injection | 1.72 ml |
| | | 2.00 ml |

### Industrial Applicability

The present invention provides an antitussive which comprises, as an active ingredient, a tricyclic compound or a pharmaceutically acceptable salt thereof.

## Claims

1. An antitussive which comprises, as an active ingredient, a tricyclic compound represented by Formula (I) 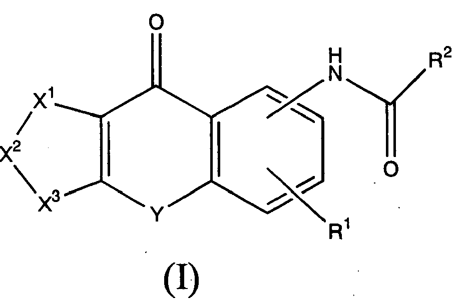
{wherein R¹ represents a hydrogen atom, substituted or unsubstituted lower alkyl, substituted or unsubstituted lower alkoxy or halogen,
X¹-X²-X³ represents CR⁵=CR⁶-CR⁷=CR⁸ [wherein R⁵, R⁶, R⁷ and R⁸ may be the same or different and each represents a hydrogen atom, substituted or unsubstituted lower alkyl, hydroxy, substituted or unsubstituted lower alkoxy, nitro, amino, mono(lower alkyl)-substituted amino, di(lower alkyl)-substituted amino, substituted or unsubstituted lower alkanoylamino or halogen], N(O)ₘ=CR⁶-CR⁷=CR⁸ (wherein R⁶, R⁷ and R⁸ have the same meanings as defined above, respectively and m represents 0 or 1), CR⁵=CR⁶-N(O)ₘ=CR⁸ (wherein R⁵, R⁶, R⁸ and m have the same meanings as defined above, respectively), CR⁵=CR⁶CR⁷=N(O)ₘ (wherein R⁵, R⁶, R⁷ and m have the same meanings as def ined above, respectively), CR⁵=CR⁶-O (wherein R⁵ and R⁶ have the same meanings as defined above, respectively), CR⁵=CR⁶-S (wherein R⁵ and R⁶ have the same meanings as defined above, respectively), O-CR⁷=CR⁸ (wherein R⁷ and R⁸ have the same meanings as defined above, respectively), S-CR⁷=CR⁸ (wherein R⁷ and R⁸ have the same meanings as defined above, respectively) or O-CR⁷=N (wherein R⁷ has the same meaning as defined above),
Y represents -CH₂S-, -CH₂SO-, -CH₂SO₂-, -CH₂O-, -CH=CH-, -(CH₂)ₚ-(wherein p represents an integer of 0 to 2), -SCH₂-, -SOCH₂-, -SO₂CH₂- or -OCH₂-, and
R² represents a hydrogen atom, substituted or unsubstituted lower alkyl, substituted or unsubstituted lower alkenyl, substituted or unsubstituted lower alkoxy, amino, mono(substituted or unsubstituted lower alkyl)-substituted amino, di(substituted or unsubstituted lower alkyl)-substituted amino, substituted or unsubstituted aryl, substituted or unsubstituted heteroaryl, substituted or unsubstituted aralkylamino, substituted or unsubstituted arylamino, or a substituted or unsubstituted heterocyclic group} or a pharmaceutically acceptable salt thereof.

2. An antitussive which comprises, as an active ingredient, a tricyclic compound represented by Formula (Ia) 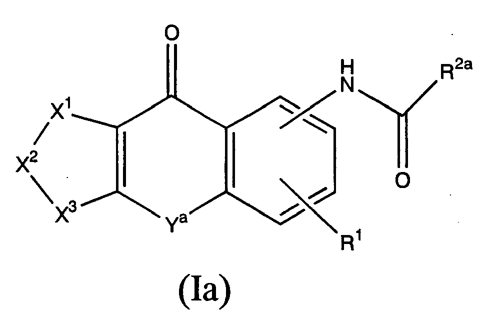
[wherein R¹ and X¹-X²-X³ have the same meanings as defined above, respectively,
Y^{a} represents -CH₂SO₂-, -SCH₂-, -SOCH₂-, -SO₂CH₂- or -OCH₂- and when Y^{a} is -CH₂SO₂-, -SCH₂-, -SOCH₂- or -SO₂CH₂-,
R^{2a} represents a hydrogen atom, substituted or unsubstituted lower alkyl, substituted or unsubstituted lower alkenyl, substituted or unsubstituted lower alkoxy, amino, mono(substituted or unsubstituted lower alkyl)-substituted amino, di(substituted or unsubstituted lower alkyl)-substituted amino, substituted or unsubstituted aryl, substituted or unsubstituted heteroaryl, substituted or unsubstituted aralkylamino, substituted or unsubstituted arylamino, a substituted or unsubstituted heteroalicyclic group, or a substituted or unsubstituted nitrogen-containing heterocyclic group and
when Y^{a} is -OCH₂-,
R^{2a} represents a hydrogen atom, trifluoromethyl, substituted or unsubstituted lower alkenyl, substituted or unsubstituted lower alkoxy, amino, mono(substituted or unsubstituted lower alkyl)-substituted amino, di(substituted or unsubstituted lower alkyl)-substituted amino, substituted or unsubstituted aryl, substituted or unsubstituted heteroaryl, substituted or unsubstituted aralkylamino, substituted or unsubstituted arylamino, a substituted or unsubstituted heteroalicyclic group, a substituted or unsubstituted nitrogen-containing heterocyclic group, or Formula (II) 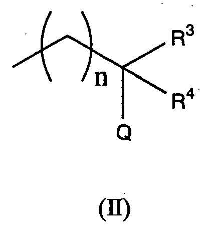
(wherein n is 0 or 1; R³ and R⁴ may be the same or different and represents a hydrogen atom, substituted or unsubstituted lower alkyl, substituted or unsubstituted cycloalkyl, substituted or unsubstituted aryl, or substituted or unsubstituted aralkyl, or R³ and R⁴ may be combined together with the adjacent carbon atom thereto to form cycloalkyl; and Q represents hydroxy, substituted or unsubstituted lower alkoxy, amino or halogen)] or a pharmaceutically acceptable salt thereof.

3. The antitussive according to Claim 2, wherein Y^{a} is -CH₂SO₂-, -SCH₂-, -SOCH₂- or -SO₂CH₂-.

4. The antitussive according to Claim 2, wherein Y^{a} is -OCH₂-.

5. The antitussive according to any of Claims 2 to 4, wherein R¹ is a hydrogen atom, substituted or unsubstituted lower alkoxy or halogen.

6. The antitussive according to any of Claims 2 to 4, wherein R¹ is a hydrogen atom.

7. The antitussive according to any of Claims 2, 5 and 6, wherein Y^{a} is -CH₂SO₂-, -SO₂CH₂- or -OCH₂-.

8. The antitussive according to any of Claims 2, 5 and 6, wherein Y^{a} is -CH₂SO₂- or -SO₂CH₂-.

9. The antitussive according to any of Claims 2, 5 and 6, wherein Y^{a} is -CH₂SO₂-.

10. The antitussive according to any of Claims 2 to 9, wherein X¹-X²-X³ is S-CR⁷=CR⁸ (wherein R⁷ and R⁸ have the same meanings as defined above, respectively).

11. The antitussive according to any of Claims 2 to 9, wherein X¹-X²-X³ is CR⁵=CR⁶-CR⁷=CR⁸ (wherein R⁵, R⁶, R⁷ and R⁸ have the same meanings as defined above, respectively).

12. The antitussive according to any of Claims 2 to 11, wherein R^{2a} is Formula (II) 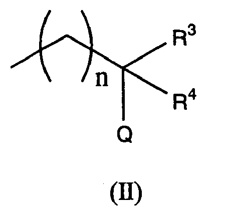
(wherein n, R³, R⁴ and Q have the same meanings as defined above, respectively).

13. The antitussive according to Claim 12, wherein n is 0.

14. The antitussive according to Claim 13, wherein R³ is methyl, R⁴ is trifluoromethyl, and Q is hydroxy.

15. The antitussive according to Claim 2, wherein R¹ is a hydrogen atom, Y^{a} is -CH₂SO₂-, X¹-X²-X³ is S-CR⁷=CR⁸ (wherein R⁷ and R⁸ have the same meanings as defined above, respectively), and R² is Formula (III)

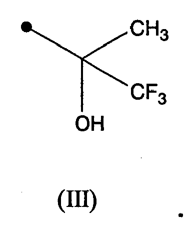

16. An antitussive which comprises, as an active ingredient, a tricyclic compound represented by Formula (Ib) 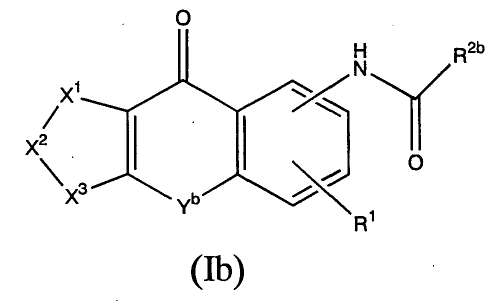
[wherein R¹ and X¹-X²-X³ have the same meanings as defined above, respectively,
Y^{b} represents -CH₂O-, -CH₂S-, -CH₂SO-, -CH=CH- or -(CH₂)_{P}-(wherein p has the same meaning as defined above) and
R^{2b} represents Formula (III) 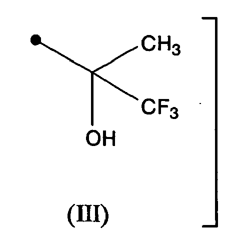
or a pharmaceutically acceptable salt thereof.

17. The antitussive according to Claim 16, wherein X¹-X²-X³ is CR⁵=CR⁶-CR⁷=CR⁸ (wherein R⁵, R⁶, R⁷ and R⁸ have the same meanings as defined above, respectively) or CR⁵=CR⁶-CR⁷=N (wherein R⁵, R⁶ and R⁷ have the same meanings as defined above, respectively).

18. The antitussive according to Claim 16, wherein X¹-X²-X³ is CR⁵=CR⁶-O (wherein R⁵ and R⁶ have the same meanings as defined above, respectively) or CR⁵=CR⁶-S (wherein R⁵ and R⁶ have the same meanings as defined above, respectively).

19. The antitussive according to Claim 16, wherein X¹-X²-X³ is O-CR⁷=CR⁸ (wherein R⁷ and R⁸ have the same meanings as defined above, respectively) or S-CR⁷=CR⁸ (wherein R⁷ and R⁸ have the same meanings as defined above, respectively).

20. The antitussive according to any of Claims 16 to 19, wherein Y^{b} is -CH₂O-.

21. The antitussive according to any of Claims 16 to 19, wherein Y^{b} is -(CH₂)ₚ- (wherein p has the same meaning as defined above).

22. The antitussive according to Claim 21, wherein p is 0.

23. The antitussive according to Claim 21, wherein p is 2.

24. The antitussive according to any of Claims 16 to 19, wherein Y^{b} is -CH=CH-.

25. The antitussive according to any of Claims 16 to 19, wherein Y^{b} is -CH₂S- or -CH₂SO-.

26. A method for alleviation of a cough, which comprises a step of administering an effective amount of the tricyclic compound or the pharmaceutically acceptable salt thereof described in any of Claims 1 to 25.

27. Use of the tricyclic compound or the pharmaceutically acceptable salt thereof described in any of Claims 1 to 25 for the manufacture of an antitussive.
